# EUROPEAN PATENT APPLICATION

(11) **EP 3 875 956 A1**
(43) Date of publication of application: **08.09.2021**
(21) Application number: 19878672.5
(22) Date of filing: 30.10.2019
(51) Int. Cl.: G01N 33/48, G01N 33/49

(54) **QUANTITATIVE FLOW CYTOMETRY**

(30) Priority: 31.10.2018 JP 2018205368
(71) Applicant: Takeda Pharmaceutical Company Limited, Osaka-shi, Osaka 541-0045 (JP)
(72) Inventor: YAMAMOTO, Shunsuke, Fujisawa-shi, Kanagawa 251-0012 (JP); MATSUMOTO, Shinichi, Fujisawa-shi, Kanagawa 251-0012 (JP); SHIMIZU, Hisao, Fujisawa-shi, Kanagawa 251-0012 (JP); HIRABAYASHI, Hideki, Fujisawa-shi, Kanagawa 251-0012 (JP)
(74) Representative: Jones, Nicholas Andrew
(86) International application number: PCT/JP2019/042470
(87) International publication number: WO 2020/090852

(57) **Abstract**

The present disclosure relates to a method for measuring the cell concentration in a sample, comprising the following steps:
(1) measuring the concentrations of cells in standard samples each having a known concentration using a flow cytometer;
(2) preparing a calibration curve based on the values measured in step (1) and the known concentrations;
(3) measuring the concentration of cells in a sample having an unknown concentration using a flow cytometer; and
(4) determining the concentration of cells from the value measured in step (3) based on the calibration curve prepared in step (2).

## Description

### Technical Field

The present invention relates to a method for measuring the cell concentration in a sample using a flow cytometer.

### Background Art

Flow cytometry is a method for measuring the size of individual cells in a cell population, DNA content, and distribution of expression of membrane antigens based on a principle in which particles in a water stream are irradiated with excitation light to measure fluorescence emitted from individual particles. A measurement device used for flow cytometry is called a flow cytometer.

Patent Literature (PTL) 1 reports a method for determining the absolute count of cells using flow cytometry. The method described in PTL 1 comprises the following steps:
(a) adding a sample to a tube containing a diluent comprising one or more cell markers so that the cells in each population within the sample are labelled with the cell markers and a known number of fluorescent microparticles;
(b) setting a fluorescence trigger to include essentially all of the microparticles and cells in the populations to be counted;
(c) setting one or more fluorescence gates to distinguish between each of the cell markers and the microparticles;
(d) counting the number of cells from step (c) that meet or exceed the fluorescence trigger; and
(e) calculating the number of cells per microparticle for each fluorescence gate from step (d) and multiplying each by the concentration of microparticles, resulting in the absolute count of cells per unit volume.

A known number of fluorescent microparticles correspond to beads, which are internal standards. In the method of PTL 1, a known number of beads are placed in a sample tube and run on a flow cytometer together with target cells for simultaneous measurement. Thereafter, the actual measurement value of target cells is normalized by the actual measurement value of beads to thereby reduce measurement variation among samples (improve precision).

However, PTL 1 does not take into consideration whether the target cells are not lost in the sample treatment process, or whether all of the target cells can be measured using the flow cytometer. Accordingly, although the method of PTL 1 relates to "a method for measuring the absolute count of cells," accuracy is not guaranteed.

### Citation List

### Patent Literature

PTL 1: JP1992-252957A

### Summary of Invention

### Technical Problem

As described above, although the method of PTL 1 can control precision, the accuracy cannot be evaluated because the recovery rate of target cells is not taken into consideration. In order to evaluate accuracy, the recovery rate of target cells must be obtained; and to obtain the recovery rate, a calibration curve must be made. However, PTL 1 nowhere discloses the recovery rate of a standard material, the calibration curve, and the evaluation of accuracy.

The present invention aims to provide a method for measuring the concentration of cells in a sample using a flow cytometer, which enables highly accurate quantification of the number of cells.

### Solution to Problem

As a result of extensive research to achieve the above object, the present inventors found the following. Based on known concentrations and the concentrations of cells in standard samples measured using a flow cytometer, a calibration curve is made; and the concentration of cells is determined using the calibration curve based on the measurement values of cell concentrations in the sample measured using the flow cytometer; thus, the number of cells can be quantified with high accuracy.

The present invention was accomplished based on these findings, and provides the following method for measuring the concentration of cells in a sample.

Item 1. A method for measuring the concentration of cells in a sample, comprising the following steps:
   (1) measuring the concentrations of cells in standard samples each having a known concentration using a flow cytometer;
   (2) preparing a calibration curve based on the values measured in step (1) and the known concentrations;
   (3) measuring the concentration of cells in a sample having an unknown concentration using a flow cytometer; and
   (4) determining the concentration of cells from the value measured in step (3) using the calibration curve prepared in step (2) .
Item 2. The method according to Item 1, wherein two or more standard samples each having a known concentration are used.
Item 3. The method according to Item 1 or 2, wherein one or more standard samples are used for each known concentration.
Item 4. The method according to any one of Items 1 to 3, wherein in step (2), values that fall within an error of ±30% relative to the known concentrations are used to prepare the calibration curve.
Item 5. The method according to any one of Items 1 to 4, wherein the accuracy of the values measured in step (1) relative to the known concentrations is within ±30% for all values.
Item 6. The method according to any one of Items 1 to 5, wherein the sample is blood.
Item 7. The method according to any one of Items 1 to 6, wherein the cells are T cells.

### Advantageous Effects of Invention

The method for measuring the concentration of cells according to the present invention enables highly accurate quantification using a flow cytometer.

### Description of Embodiments

Embodiments of the present invention are described in detail below.

In this specification, the term "comprise" includes the meaning "essentially consist of" and the meaning "consist of."

The "accuracy" used herein is the degree of accuracy indicating that the value is close to the true value. Specifically, the "accuracy" becomes higher as the average value is closer to the true value.

The "precision" used herein is a scale indicating that there is a little variation among the values obtained in multiple measurements.

The method for measuring the concentration of cells in a sample according to the present invention includes the following steps:
(1) measuring the concentrations of cells in standard samples each having a known concentration using a flow cytometer;
(2) preparing a calibration curve based on the values measured in step (1) and the known concentrations;
(3) measuring the concentration of cells in a sample having an unknown concentration using a flow cytometer; and
(4) determining the concentration of cells from the value measured in step (3) using the calibration curve prepared in step (2) .

In the present invention, the sample in which the concentration of cells is measured is not particularly limited, as long as it can be measured by a flow cytometer. Examples include blood, tumor, cerebrospinal fluid, liver, lung, spleen, thymus, bone marrow, and the like.

Before the measurement using a flow cytometer, the sample can be suitably subjected to various pretreatments. For example, if blood is used as a sample, a pretreatment such as hemolysis can be performed before measurement. Such a hemolytic treatment can be performed by a known method. As a method of hemolyzing red blood cells, a surfactant can be added. Other pretreatments include cell fixation. Examples of cell fixation agents used for such cell fixation include paraformaldehyde, glutaraldehyde, methanol, and the like.

In the present invention, cells whose concentration is measured are not particularly limited, as long as the number of cells can be measured by a flow cytometer. Examples include neural stem cells, hematopoietic stem cells, mesenchymal stem cells, dental pulp stem cells, ES cells, induced pluripotent stem (iPS) cells, tissue precursor cells, blood cells (erythrocytes, leukocytes, and platelet), epithelial cells, endothelial cells, muscle cells, fibroblast cells, smooth muscle cells, hair cells, hepatocytes, gastric mucosa cells, intestinal cells, spleen cells, pancreatic cells, brain cells, lung cells, kidney cells, adipocytes, cardiomyocytes, osteoblasts, neurons, vascular endothelial cells, bone cells, osteoclasts, cartilage cells, cartilage progenitor cells, corneal cells, retinal cells, dendritic cells, primary cultured cells, passive cells, and established cells. Examples of the leukocytes include monocytes, lymphocytes (T cells, B cells, and NK cells), dendritic cells, macrophages, eosinophils, neutrophils, and basophils. Examples of the T cells include helper T cells, cytotoxic T cells, regulatory T cells, suppressor T cells, and CAR-T cells (chimeric antigen receptor T cells).

The flow cytometer used in the present invention is not particularly limited, and various commercially available products can be used. Examples of such commercially available products include those produced by Beckman Coulter Inc. (e.g., Gallios, Navios, Navios EX, CytoFLEX, CytoFLEX S, CytoFLEX LX, and Cytomics FC 500); those produced by BD Biosciences (e.g., BD FACSCanto™ II flow cytometer, BD FACSVerse™ flow cytometer, BD FACSLyric™ flow cytometer, BD LSRFortessa™ flow cytometer, BD LSRFortessa™ X-20 flow cytometer); those produced by Thermo Fisher Scientific Inc. (e.g., Attune flow cytometer); those produced by Sony Corporation (e.g., SA3800 and SP6800Z); and the like. Measurement of the cell concentration with a flow cytometer can be performed according to a known method. For example, a manual of a flow cytometer manufacturer can be used.

In flow cytometry, target cells can be detected based on the cell size (forward scatter: FSC), cell density (side scatter: SSC), and cell surface antigen (fluorescence by a fluorescently labeled antibody).

In the measurement using a flow cytometer, a fluorescently labeled antibody capable of detecting target cells can be suitably selected and used. Examples of such a fluorescently labeled antibody include those that specifically bind to various CD antigens. Various fluorescently labeled antibodies are commercially available, and commercially available products can be used. Fluorescently labeled antibodies can be obtained by preparing antibodies, and fluorescently labeling them. As a compound for fluorescently labeling antibodies, a wide variety of known fluorescent labeling compounds can be used.

The measurement using flow cytometry can also use fluorescent beads, which are internal standards (see PTL 1). By placing such beads having a known number in a sample tube, and taking them into a flow cytometer together with target cells for simultaneous measurement, the measurement value of target cells are normalized with the measurement value of beads; i.e., the measurement value of target cells are corrected with the known number of beads in the sample tube. Thereby, the number of target cells that can be present in the sample tube can be calculated.

### Step (1)

In step (1), in order to prepare a calibration curve, the concentrations of cells in standard samples each having a known concentration are measured using a flow cytometer.

The number of concentrations of the standard samples used in step (1) is not particularly limited, as long as a calibration curve can be made. It is, for example, two or more, preferably three or more, more preferably four or more, even more preferably five or more, and particularly preferably six or more. The upper limit is, for example, 30.

As a method for preparing a standard sample, a wide variety of methods that are capable of adjusting the cell concentration can be used. Examples include a method in which a hemocytometer is used to visually count the number of cells with a microscope. Once one standard sample can be prepared, the other standard samples can be made by diluting the prepared standard sample stepwise.

The number of standard samples for each known concentration used in step (1) is not particularly limited; and it is, for example, one or more, preferably two or more, more preferably three or more, even more preferably four or more, particularly preferably five or more, and further more preferably six or more. The upper limit is, for example, 90.

The accuracy of the values measured in step (1) relative to the known concentrations is preferably within ±30% for all values, more preferably within ±25% for all values, even more preferably within ±20% for all values, and particularly preferably within ±15% for all values. The "accuracy" herein is a value in which a difference between the average of the measurement values and each known concentration is expressed by percentage relative to the known concentration (relative error). Use of the measurement values having such accuracy for the preparation of a calibration curve enables more accurate quantification of cell concentration.

The precision of the values measured in step (1) relative to the known concentrations is preferably within ±30% for all values, more preferably within ±25% for all values, even more preferably within ±20% for all values, and particularly preferably within ±15% for all values. The value of "precision" herein means the coefficient of variation (CV). Use of measurement values having such precision for the preparation of a calibration curve enables more accurate quantification of cell concentration.

### Step (2)

In step (2), a calibration curve is prepared based on the values measured in step (1) and the known concentrations.

The calibration curve can be prepared by using software or the like according to a known method, such as a least-squares method. The closer the correlation coefficient of the prepared calibration curve is 1, the more desirable it is.

By thus preparing the calibration curve, the recovery rate of target cells in the sample preparation process can be obtained. This consequently enables the evaluation of accuracy.

In step (2) (especially when the slope of the calibration curve is approximately 1), it is desirable to use measurement values that fall within an error of ±30% (preferably within ±25%, more preferably within ±20%, and even more preferably within ±15%) relative to the known concentration for the preparation of a calibration curve. Use of measurement values having such an error range for the preparation of the calibration curve enables more accurate quantification of cell concentration.

When the slope of the calibration curve does not equal 1 (for example, when the recovery rate of cells does not equal 100%), it is desirable to calculate the number of cells from the measurement value using the formula of the calibration curve, and verify the accuracy of the calculated value relative to the known concentration.

### Step (3)

In step (3), the concentration of cells in a sample having an unknown concentration is measured using a flow cytometer.

### Step (4)

In step (4), using the calibration curve prepared in step (2), the concentration of cells is determined from the value measured in step (3).

Such determination of cell concentration using a calibration curve enables highly accurate quantification using a flow cytometer.

### Examples

The following examples are given to illustrate the present invention in more detail. However, the present invention is not limited to these examples.

### Method

50 µL of C.B-17 SCID mouse blood was used as a specimen. 10, 100, 1,000, and 10,000 cells of human T cells (peripheral blood T cells, Cryo-T8: Human CD8⁺ Negatively Selected (5-8M cells), produced by Precision Bioservices Inc.) were individually added to the specimen to prepare samples, and a sample containing no human T cell solution was also prepared. These samples were individually mixed with a BD Tritest™ CD3/CD8/CD45 reagent (produced by BD Biosciences) in commercially available tubes (TruCOUNT Tubes, produced by BD Biosciences). This reagent contains fluorescein isothiocyanate (FITC)-labeled anti-CD3 antibody (clone SK7), phycoerythrin (PE)-labeled anti-CD8 antibody (clone SK1), and peridinin chlorophyll protein (PerCP)-labeled anti-CD45 antibody (clone 2D1).

After the samples were allowed to stand at room temperature for 15 minutes, blood was lysed using a BD FACS™ lysing solution (BD Biosciences) to fix the cells. The samples were allowed to stand for another 15 minutes, and then subjected to flow cytometric quantification. The flow cytometry measurement was performed using a BD FACSLyric™ flow cytometer (produced by BD Biosciences). For analysis, FlowJo software (Ver. 10, BD Biosciences) was used, and human CD45/CD3/CD8 positive cells were detected as target cells. At each concentration, the samples were prepared at n=3 and measured.

### Results

Table 1 shows the results of preparation of a calibration curve. The linearity of the calibration curve was excellent, and the recovery rate was approximately 100%. The intra-day and inter-day accuracy (relative error) and precision (coefficient of variation) were also excellent. The lower limit of quantification was 30 cells/50 µL.

**Table 1**

| | 1st day | | | 2nd day | | | 3rd day | | |
|---|---|---|---|---|---|---|---|---|---|
| Nominal concentration (cells/50 µL,) | Mean observed concentration (cells/50 µL) | Relative error (%) | Coefficient of variation (%) | Mean observed concentration (cells/50 µL) | Relative error (%) | Coefficient of variation (%) | Mean observed concentration (cells/50 µL) | Relative error (%) | Coefficient of variation (%) |
| 30 | 36 | -19.0 | 23.6 | 32 | -5.3 | 17.7 | 25 | 16.2 | 7.7 |
| 100 | 88 | 12.5 | 7.0 | 92 | 7.8 | 4.5 | 105 | -5.4 | 8.8 |
| 300 | 309 | -3.0 | 10.7 | 278 | 7.2 | 15.6 | 287 | 4.4 | 2.1 |
| 1000 | 1005 | -0.5 | 6.0 | 881 | 11.9 | 5.0 | 1003 | -0.3 | 4.0 |
| 3000 | 2875 | 4.2 | 3.3 | 2857 | 4.8 | 2.3 | 3156 | -5.2 | 4.1 |
| 10000 | 10171 | -1.7 | 1.9 | 9502 | 5.0 | 2.5 | 10479 | -4.8 | 5.1 |
| Slope | 1.015 | | | 0.951 | | | 1.049 | | |
| y-intercept | -24.0 | | | -12.0 | | | -12.5 | | |
| r² value | 1.000 | | | 0.999 | | | 1.000 | | |

## Claims

1. A method for measuring the concentration of cells in a sample, comprising the following steps:
(1) measuring the concentrations of cells in standard samples each having a known concentration using a flow cytometer;
(2) preparing a calibration curve based on the values measured in step (1) and the known concentrations;
(3) measuring the concentration of cells in a sample having an unknown concentration using a flow cytometer; and
(4) determining the concentration of cells from the value measured in step (3) using the calibration curve prepared in step (2) .

2. The method according to claim 1, wherein two or more standard samples each having a known concentration are used.

3. The method according to claim 1 or 2, wherein one or more standard samples are used for each known concentration.

4. The method according to any one of claims 1 to 3,
wherein in step (2), values that fall within an error of ±30% relative to the known concentrations are used to prepare the calibration curve.

5. The method according to any one of claims 1 to 4,
wherein the accuracy of the values measured in step (1) relative to the known concentrations is within ±30% for all values.

6. The method according to any one of claims 1 to 5, wherein the sample is blood.

7. The method according to any one of claims 1 to 6, wherein the cells are T cells.
